Europäisches Patentamt

European Patent Office

Office européen des brevets

(11) Publication number: **0 452 578 B1**

# EUROPEAN PATENT SPECIFICATION

(45) Date of publication of patent specification: **15.03.95**   (51) Int. Cl.6: **A61B 5/022**

(21) Application number: **90304161.4**

(22) Date of filing: **18.04.90**

(54) **Pulse wave detecting apparatus.**

(43) Date of publication of application:
**23.10.91 Bulletin 91/43**

(45) Publication of the grant of the patent:
**15.03.95 Bulletin 95/11**

(84) Designated Contracting States:
**DE FR GB IT NL SE**

(56) References cited:
**WO-A-88/03387**
**JP-A- 2 109 540**
**US-A- 4 836 213**

(73) Proprietor: **COLIN CORPORATION**
**2007-1, Hayashi**
**Komaki-shi**
**Aichi-ken (JP)**

(72) Inventor: **Niwa, Minoru, c/o Colin Electronics**
**Co. Ltd.**
**2007-1, Hayashi**
**Komaki-shi,**
**Aichi-ken (JP)**

(74) Representative: **Senior, Alan Murray et al**
**J.A. KEMP & CO.,**
**14 South Square,**
**Gray's Inn**
**London WC1R 5LX (GB)**

EP 0 452 578 B1

## Description

## BACKGROUND OF THE INVENTION

Field of the Art

The present invention relates to an apparatus for detecting a pulse wave produced from an arterial vessel of a subject.

Related Art Statement

There is known a pulse wave detecting apparatus including (1) a pulse wave sensor having a press surface on which one or more pressure sensing elements are provided, the pulse wave sensor being pressed against an arterial vessel of a subject via a body surface above the arterial vessel so that the one or more pressure sensing elements detect a pulse wave produced from the arterial vessel of the subject, (2) pressing means for pressing the pulse wave sensor against the arterial vessel via the body surface, while varying the pressing force over a predetermined force range, and (3) means for determining an optimum, pulse wave sensor pressing force based on the pulse wave (pulses of the pulse wave) which is detected by the one or more pressure sensing elements while the pressing force is varied, and (4) means for obtaining a pulse wave of the subject through the pulse wave sensor pressed with the thus determined optimum pressing force. The conventional apparatus determines as the optimum pulse wave sensor pressing force a pressing force at which the pulse wave sensor (one or more pressure sensing elements) detects a pulse of the pulse wave whose amplitude is maximum, namely, the greatest of the amplitudes of the pulses detected by the pulse wave sensor in the process of varying the pressing force.

However, generally the thus determined optimum pressing force does not fall to around the middle value of a pressing force range which covers pressing force values at which a pulse wave having a sufficiently great amplitude is detected. Accordingly, in the event that the pulse wave sensor pressing force is changed from the optimum pressing force to a greater or smaller value due to physical motion of the subject or other causes, the conventional apparatus may fail to detect a pulse wave having a sufficiently great amplitude.

WO-A-88/03387 discloses a pulse wave detecting means as set out in the preamble of claim 1.

It is therefore an object of the present invention to provide a pulse wave detecting apparatus capable of detecting a pulse wave having a sufficiently great amplitude irrespective of change in the pulse wave sensor pressing force.

The present invention provides: a pulse wave detecting apparatus for detecting a pulse wave produced from an arterial vessel of a subject, comprising: a pulse wave sensor having a press surface on which at least one pressure sensing element is provided, said pulse wave sensor to be pressed against an arterial vessel of a subject via a body surface above said arterial vessel so that said at least one pressure sensing element detects a pulse wave produced from said arterial vessel of said subject;

pressing means for pressing said pulse wave sensor against said arterial vessel via said body surface, while varying the pressing force over a predetermined force range;

amplitude determining means for determining the amplitude of each of the pulses of the pulse wave which are detected by said at least one pressure sensing element while the pressing force of said pressing means is varied;

minimum value determining means for determining a minimum value of said each pulse of the pulse wave detected by said at least one pressure sensing element;

first and second pressing force range determining means

optimum pressing force determining means for determining an optimum value of said pressing force; and

means for adjusting said pressing means to said optimum value of said pressing force and obtaining a pulse wave of said subject through said pulse wave sensor pressed against said arterial vessel with the optimum pressing force, the apparatus being characterised in that the first pressing force range determining means is adapted to determine, based on the pulse amplitudes determined by said amplitude determining means, a first force range of said pressing force within which the change of said pulse amplitudes is smaller than a first predetermined value, said first force range including a point at which a rate of change of said pulse amplitudes with respect to said pressing force is zero;

the second pressing force range determining means is adapted to determine, based on the pulse minimum values determined by said minimum value determining means, a second force range of said pressing force within which the change of said pulse minimum values is smaller than a second predetermined value, said second force range including a point at which a rate of change of said pulse minimum values with respect to said pressing force is minimum;

optimum pressing force range determining means is provided for determining, as an optimum force range of said pressing force, an overlap portion of said first and second force ranges in the

2

event that neither said first force range nor said second force range is zero; and

the optimum pressing force determining means is adapted to determine said optimum value of said pressing force such that said optimum value falls within said optimum force range.

In the pulse wave detecting apparatus constructed as described above, the optimum force range of the pulse wave sensor pressing force is determined based on the first force range within which the pulse amplitudes are generally constant and which covers a pressing force at which the pulse wave sensor has detected a pulse having a maximum amplitude, and the second force range within which the pulse minimum values is generally constant. So long as the pulse wave sensor is pressed with a pressing force falling within the optimum force range, the pulse wave sensor detects a pulse wave whose amplitude is sufficiently great and generally constant. Accordingly, even in the event that the pulse wave sensor pressing force is changed due to physical motion of the subject or the like during the pulse wave detection, the present apparatus continues to detect a pulse wave having a sufficiently great amplitude.

The above and optional objects, features and advantages of the present invention will be better understood by reading the following detailed description of the presently preferred embodiment of the invention when considered in connection with the accompanying drawings in which:

Fig. 1 is a view illustrating the circuit of the pulse wave detecting apparatus of the present invention;

Fig. 2 is a view of a pulse wave sensor of the apparatus of Fig. 1, as viewed from the side of a body surface of a subject;

Fig. 3 is a flow chart according to which the apparatus of Fig. 1 is operated; and

Fig. 4 is a graph showing a curve representative of change of the pulse amplitudes with respect to the pulse wave sensor pressing forces, and a curve representative of change of the pulse minimum values with respect to the pulse wave sensor pressing forces.

## DETAILED DESCRIPTION OF THE PREFERRED EMBODIMENT

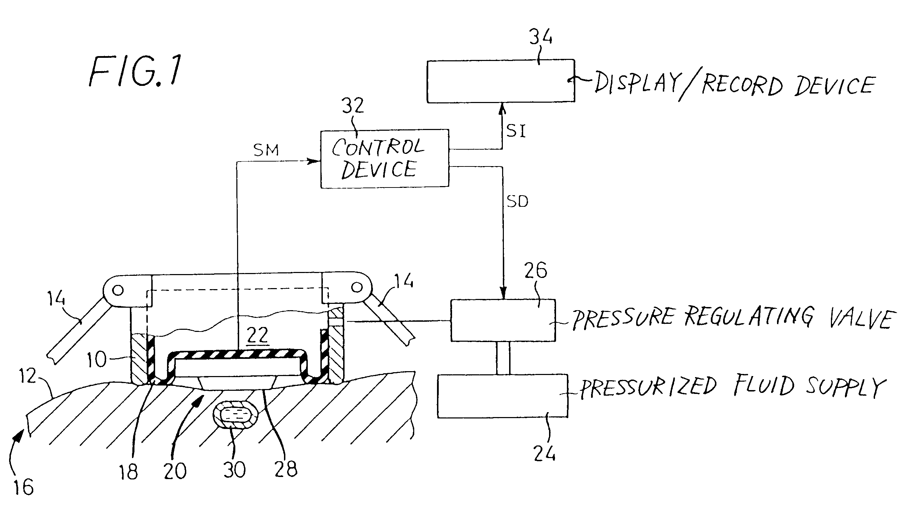

Referring first to Fig. 1 there is illustrated a pulse wave detecting apparatus embodying the present invention. In the figure reference numeral 10 designates a cylindrical housing having a bottom end and an open end. The housing 10 is detachably set on a body surface 12 of a wrist 16 of a subject with the help of a band 14, with the open end of the housing 10 being opposed to the body surface 12. A diaphragm 18 is fluid-tightly secured to inner surfaces of the housing 10, and cooperates with the housing 10 to define a fluid-tight pressure chamber 22. A pulse wave sensor 20 for detecting pulse wave is secured to an outer surface of the diaphragm 18. A pressurized fluid supply 24 supplies the pressure chamber 22 with a pressurized fluid such as pressurized air. A pressure regulating valve 26 regulates the pressurized air supplied to the pressure chamber 22 and thereby adjusts an air pressure P in the pressure chamber 22. As the air pressure P in the pressure chamber 22 is increased or decreased, the diaphragm 18 is inflated or deflated and the pulse wave sensor 20 is displaced together with the diaphragm 18 relative to the housing 10, so that the pulse wave sensor 20 is advanced out of the open end of the housing 10 and is pressed against the body surface 12 with a pressing force corresponding to an air pressure P in the pressure chamber 22.

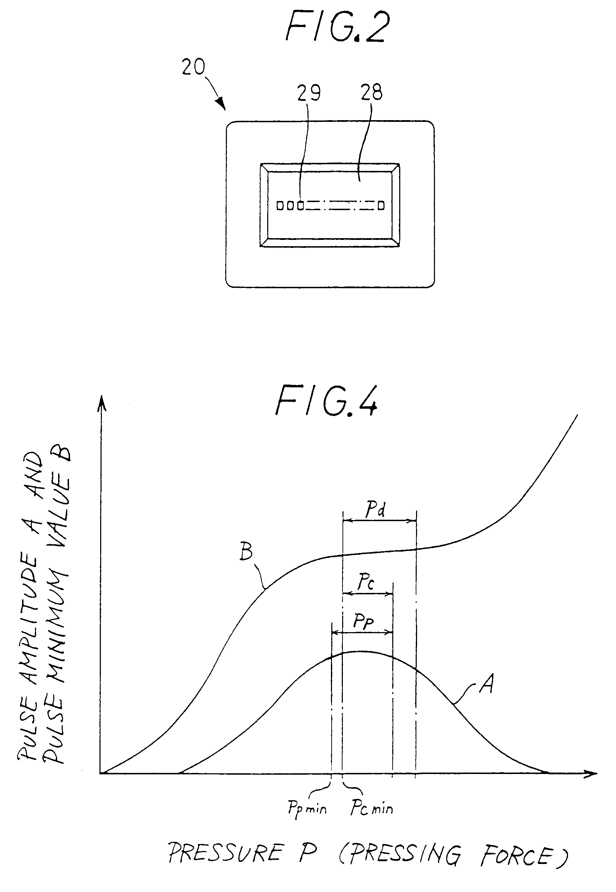

As shown in Fig. 2 the pulse wave sensor 20 is constituted by a semiconductor chip formed of, for example, monocrystalline silicon, and a multiplicity of pressure sensing elements 29, for example fifteen pressure sensing diodes, provided in a row on one surface 28 of the semiconductor chip. Thus, the chip surface 28 serves as a press surface at which the pulse wave sensor 20 is pressed against the body surface 12. With the housing 10 being attached to the wrist 16, the row of the pressure sensing elements 29 extends in a direction generally perpendicular to a radial artery 30 extending adjacent to a radius bone of the wrist 16. In other words, the pulse wave sensor 20 is pressed against the radial artery 30 via the body surface 12 such that the row of the pressure sensing elements 29 generally perpendicularly intersects the radial artery 30. In this situation, each pressure sensing element 29 detects an oscillatory pressure wave, namely, pressure pulse wave (hereinafter, abbreviated to "pulse wave") transmitted thereto from the radial artery 30 via the body surface 12. The pulse wave consists of successive pulses, and the pulses are produced from the radial artery 30 in synchronization with heartbeat of the subject. Each of the pressure sensing elements 29 converts the detected pulse wave to an electric signal (hereinafter, referred to as "pulse wave signal") SM, and supplies the pulse wave signal SM to a control device 32.

The control device 32 includes a microcomputer, and supplies a drive signal SD to the pressure regulating valve 26 according to pre-stored programs so as to adjust the air pressure P in the pressure chamber 22 in the housing 10. The control device 32 stores pulse wave signals SM supplied from the pressure sensing elements 29, while commanding the pressure regulating valve 26 to increase the air pressure P at a constant, low rate. Based on the stored pulse wave signals SM the control device 32 selects an optimum pressure sensing element 29A out of the multiplicity of pressure sensing elements 29 and determines an optimum pressing force Pa with which the pulse wave sensor 20 (optimum pressure sensing element 29A) is to be pressed against the radial artery 30. With the pulse wave sensor 20 being pressed with the optimum pressing force Pa, the control device 32 is supplied with a pulse wave signal SM from the optimum pressure sensing element 29A, and supplies a display/record signal SI representative of the pulse wave, to a display/record device 34. Thus, the display/record device 34 displays and records the pulse wave detected by the optimum pressure sensing element 29A pressed with the optimum pressing force Pa.

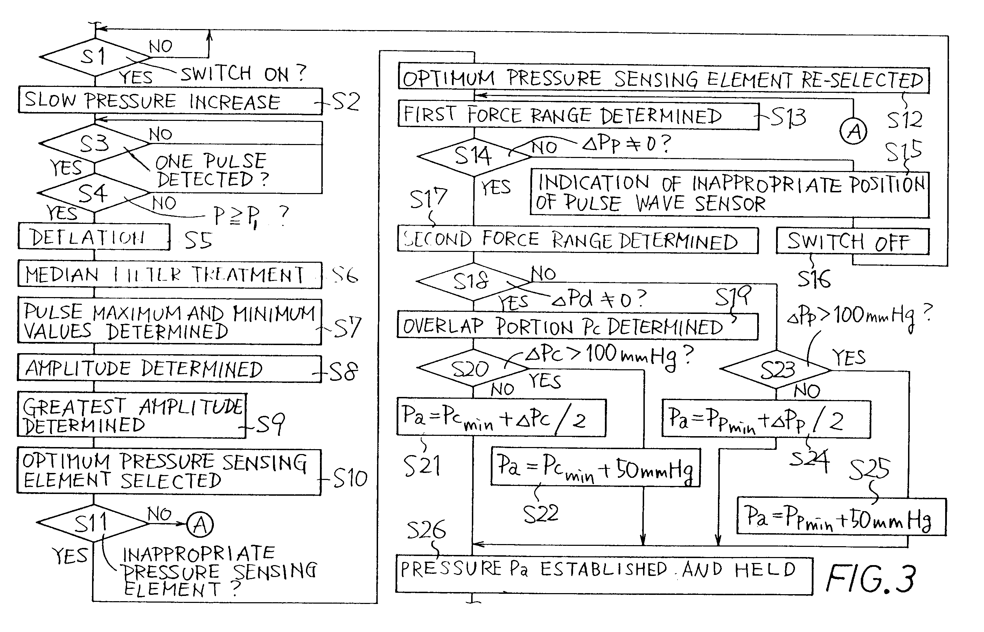

There will be described the operation of the pulse wave detecting apparatus constructed as described above, by reference to the flow chart of Fig. 3.

Upon application of electric power to the present apparatus, the control of the microcomputer of the control device 32 begins with Step S1 in which it is judged whether or not an ON/OFF switch (not shown) has been operated by an operator, namely, placed in an ON position thereof. If the judgement in Step S1 is negative (NO), Step S1 is repeated awaiting an operation of the ON/OFF switch. On the other hand, if it is judged in Step S1 that the ON/OFF switch is placed in the ON position thereof, namely, if the judgement in Step S1 is affirmative (YES), the control proceeds to Step S2 in which the air pressure P in the pressure chamber 22 is increased at a constant low rate, namely, slowly increased. Step S2 is followed by Step S3 in which it is judged based on the pulse wave signals SM whether or not in this slow pressure increase process the pressure sensing elements 29 have detected a length of pulse wave corresponding to one time heartbeat of the subject, namely, one pulse. If the judgement in Step S3 is negative, Step S3 is repeated. Meanwhile, if the judgement in Step S3 is affirmative, namely, if the pressure sensing elements 29 have detected one pulse of the pulse wave, the control device 32 stores pulse wave signals SM corresponding to the pulse, together with the corresponding values of the air pressure P in the pressure chamber 22. Subsequently, the control proceeds to Step S4 in which it is judged whether or not the air pressure P has exceeded a predetermined pressure level $P_1$, for example about 200 mmHg. If the judgement in Step S4 is negative, namely, if the air pressure P has not reached the level $P_1$, Steps S3 and S4 are repeated so that the control device 32 stores the pulse wave signals SM corresponding to a multiplicity of pulses of the pulse wave. On the other hand, if the judgement in Step S4 is affirmative, the control proceeds to Step S5 in which the pressurized air in the pressure chamber 22 is removed to decrease the air pressure P to atmospheric pressure.

Step S5 is followed by Step S6 in which the control device 32 applies a well-known median filter, a statistical treatment, to the stored pulse wave signals SM with respect to, for example, every five successive pulses of the signals SM, so as to remove artifact noise therefrom, namely, smooth the signals SM. Step S6 is followed by Step S7 in which the control device 32 determines a maximum and a minimum value of each of the pulses with respect to each of the filtered signals SM. Step S7 is followed by Step S8 in which the control device 32 determines an amplitude of each of the pulses based on the maximum and minimum values thereof determined in Step S7. Thus, in the present embodiment Step S7 and a portion of the control device 32 for carrying out Step S7 cooperate with each other to serve as means for determining a minimum value of each of pulses of the pulse wave detected by the pulse wave sensor, while Step S8 and a portion of the control device 32 for carrying out Step S8 cooperate with each other to serve as means for determining an amplitude of each of pulses of the pulse wave detected by the pulse wave sensor.

Subsequently, the control proceeds to Step S9 in which the control device 32 determines a maximum amplitude with respect to each of the pulse wave signals SM, namely, each of the pressure sensing elements 29. The maximum amplitude is the greatest amplitude of all the amplitudes of the pulses detected by the each pressure sensing element 29. Step S9 is followed by Step S10 in which the control device 32 selects as an optimum pressure sensing element 29A one of the pressure sensing elements 29 whose maximum amplitude is the greatest of all the maximum amplitudes of the pressure sensing elements 29. Step S10 is followed by Step S11 in which it is judged whether or not either one of the two pressure sensing elements positioned at opposite ends of the row of the pressure sensing elements 29 has been selected as the optimum pressure sensing element 29A. If the judgement in Step S10 is negative, the control proceeds to Step S13. On the other hand, if the judgement is affirmative, the control proceeds to Step S12 in which the control device 32 discards the pressure sensing element selected as the optimum pressure sensing element 29A in Step S11 and re-select as the optimum pressure sensing element 29A another pressure sensing element whose maximum amplitude is the greatest second to the maximum amplitude of the discarded pressure sensing element. Step S12 is followed by Step S13.

In Step S13 the control device 32 utilizes the amplitudes of the pulses detected by the optimum pressure sensing element 29A in the previously described slow pressure increase process, and the corresponding values of the air pressure P in the pressure chamber 22 (corresponding to pressing forces with which the pulse wave sensor 20 is pressed against the radial artery 30), for determining a first force range in which the pulse amplitudes are generally constant. The first force range includes a point at which the rate of change of the pulse amplitudes with respect to the air pressure values P (corresponding to the pulse wave sensor pressing forces) is zero. This point corresponds to a pressing force at which the optimum pulse wave sensor 29A has detected the pulse having the maximum amplitude. The first force range is determined such that the difference between the maximum pulse amplitude and each of the other pulse amplitudes is smaller than a considerably small value corresponding to, for example 7 mmHg in terms of pressure. The first force range is shown at Pp in the graph of Fig. 4, in which the pulse amplitudes are represented by a curved line A for easy understanding. In the present embodiment Step S13 and a portion of the control device 32 for carrying out Step S13 cooperate with each other to serve as means for determining the first force range of the pulse wave sensor pressing force.

Step S13 is followed by Step S14 in which it is judged whether a value $\Delta Pp$ obtained by subtracting a lower limit Ppmin of the first force range Pp from an upper limit of the same, is not zero, that is, whether or not the first force range Pp has any length, or exist, along the axis of abscissa of the graph of Fig. 4. If the judgement in Step S14 is negative (NO), namely, if the first force range Pp is zero, that means that a position of the pulse wave sensor 20 on the body surface 12 with respect to the radial artery 30 is inappropriate, and the control goes to Step S15 in which the control device 32 commands the display/record device 34 to display a predetermined indication informing the operator of this situation. Step S15 is followed by Step S16 in which the control device 32 causes the ON/OFF switch to be placed in the OFF position, thereby deactivating the present apparatus, and then the control returns to Step S1. However, since the ON/OFF switch has just been placed in the OFF position, the judgement in Step S1 is negative and Step S1 is repeated awaiting for another operation of the ON/OFF switch.

On the other hand, if the judgement in Step S14 is affirmative (YES), that is, if the first force range Pp is not zero, the control goes to Step S17 in which the control device 32 utilizes the minimum values of the pulses detected by the optimum pressure sensing element 29A, and the corresponding air pressure values P, for determining a second force range in which the pulse minimum values are generally constant. The second force range includes a point at which the rate of change of the pulse minimum values with respect to the air pressure values P is minimum. The second force range is determined such that the difference between a selected one of the pulse minimum values and each of the other pulse minimum values is smaller than a considerably small value corresponding to, for example 7 mmHg in terms of pressure. The second force range is shown at Pd in the graph of Fig. 4, in which the pulse minimum values are represented by a curved line B for easy understanding. In the present embodiment Step S17 and a portion of the control device 32 for carrying out Step S17 cooperate with each other to serve as means for determining the second force range of the pulse wave sensor pressing force.

Step S17 is followed by Step S18 in which it is judged whether a value $\Delta Pd$ obtained by subtracting a lower limit Pdmin of the second force range Pd from an upper limit of the same, is not zero, that is, whether or not the second force range Pd has any length, or exists, along the axis of abscissa of the graph of Fig. 4. If the judgement in Step S17 is affirmative (YES), that is, if the second force range Pd is not zero, the control advances to Step S19 in which the control device 32 determines as an optimum force range an overlap portion Pc of the first and second force ranges Pp and Pd. Step S19 is followed by Step S20 in which it is judged whether or not a value $\Delta Pc$ obtained by subtracting a lower limit Pcmin of the optimum force range Pc from an upper limit of the same is greater than, for example, 100 mmHg. If the judgement in Step S20 is negative, that is, if the value $\Delta Pc$ is not greater than 100 mmHg, the control goes to Step S21 in which the control device 32 determines an optimum air force Pa corresponding to an optimum, pulse wave sensor pressing force according to the following expression: $Pa = Pcmin + \Delta Pc/2$. On the other hand, if the judgement in Step S20 is affirmative, that is, if the value $\Delta Pc$ is greater than 100 mmHg, the control goes to Step S22 in which the control device 32 determines the optimum air force Pa according to the following expression: $Pa = Pcmin + 50 \ mmHg$.

Meanwhile, if the judgement in Step S18 is negative, that is, if the second force range Pd is zero, the control device 32 determines the first force range Pp as the optimum force range, and the control goes to Step S23 in which it is judged whether or not a value $\Delta Pp$ obtained by subtracting a lower limit Ppmin of the first force range Pp (optimum force range) from an upper limit of the same, is greater than, for example, 100 mmHg. If the judgement in Step S23 is negative, that is, if the value $\Delta Pc$ is not greater than 100 mmHg, the control goes to Step S24 in which the control device 32 determines the optimum air force Pa according to the following expression: Pa = Ppmin + $\Delta Pp/2$. On the other hand, if the judgement in Step S23 is affirmative, that is, if the value $\Delta Pp$ is greater than 100 mmHg, the control goes to Step S25 in which the control device 32 determines the optimum air force Pa according to the following expression: Pa = Ppmin + 50 mmHg.

In the present embodiment Steps S18 through S25 and a portion of the control device 32 for carrying out Steps S18-S25 cooperate with each other to serve as means for determining an optimum force range of the pulse wave sensor pressing force based on the first and second force ranges, and determining an optimum value of the pressing force such that the optimum value falls within the optimum force range.

After the determination of the optimum air pressure Pa (optimum value of the pulse wave sensor pressing force) in Step S21, S22, S24 or S25, the control goes to Step S26 in which the control device 32 controls the pressure regulating valve 26 to increase the air pressure P in the pressure chamber 22 to the optimum air pressure Pa and hold the air pressure Pa. As a result, the pulse wave sensor 20 is pressed against the radial artery 30 with an optimum pressing force corresponding to the optimum air pressure Pa. With the pulse wave sensor 20 being pressed with the optimum pressing force (Pa), the present apparatus obtains a pulse wave of the subject through the optimum pressure sensing element 29A, and commands the display/record device 34 to display and record the thus obtained pulse wave. The control device 32 may be adapted to determine a blood pressure of the subject based on the thus obtained pulse wave and command the display/record device 34 to display and record the thus determined blood pressure.

As emerges from the foregoing description, the present pulse wave detecting apparatus utilizes a pulse wave (pulses thereof) which is detected by the optimum pressure sensing element 29A as the pulse wave sensor pressing force is increased at a constant low rate, for determining the first force range Pp within which the pulse amplitudes are generally constant and which includes a pressing force at which the optimum pressure sensing element 29A has detected the pulse having the maximum amplitude. In addition, the present apparatus determines the second force range Pd within which the pulse minimum values are generally constant. In Step S19 of the flow chart of Fig. 3 the overlap portion Pc of the first and second force ranges Pp and Pd is determined as the optimum force range. So long as the pulse wave sensor 20 is pressed with a pressing force falling within the optimum force range, the pulse wave sensor 20 detects a pulse wave having a sufficiently great amplitude. In Step S21 the middle value of the overlap portion Pc is determined as the optimum pulse wave sensor pressing force Pa. Therefore, the optimum pressing force Pa falls between the middle value of the first force range Pp and the middle value of the second force range Pd. Thus, even if the pulse wave sensor pressing force is changed due to physical motion of the subject or the like during the pulse wave detection, the present apparatus continues to detect a pulse wave having a sufficiently great amplitude.

In the present embodiment, in Step S20 it is judged whether or not a difference $\Delta Pc$ between an upper and a lower limit of the overlap portion Pc is greater than 100 mmHg, and if the judgement in Step S20 is affirmative a value obtained by adding 50 mmHg to the lower limit Pcmin is used as the optimum pressing force Pa. This optimum pressing force Pa is smaller than the middle value of the overlap portion Pc. Thus, in the event that the overlap portion Pc is considerably wide, the present apparatus determines the optimum pressing force Pa at a relatively low value and thereby reduces discomfort of the subject due to the pressed pulse wave sensor 20.

Although in the graph of Fig. 4 the second force range Pd overlaps an upper portion of the first force range Pp, the second force range Pd may overlap a lower portion of the first force range $P_p$. In the latter case, the optimum pressing force Pa is determined at a value smaller than a pressing force at which the optimum pressure sensing element 29A has detected the maximum pulse amplitude. Thus, the present apparatus uses the optimum pressing force Pa smaller than an optimum pressing force that has conventionally been used by the known apparatus in which a pressing force at the time of detection of the maximum pulse amplitude is used as the optimum pressing force.

In the present embodiment, in Step S18 it is judged whether the second force range Pd is not zero. This is why there are some cases where the second force range Pd is zero, namely, the minimum values of the pulses detected by the optimum pressure sensing element 29A have no range within which differences between the pulse minimum values are smaller than a predetermined considerably small value. In those cases the first force range Pp is determined as the optimum force range, and the optimum pressing force Pa is determined based on the first force range Pp only. Thus, in the event that the first force range Pp is not zero, the apparatus does not fail to detect a pulse wave, even if the second force range Pd is zero.

In addition, in the present embodiment, in Step S14 it is judged whether the first force range Pp is not zero, and if the judgement is negative (NO), namely, if the first force range Pp is zero, in Step S15 the indication informing that the position of pressing of the pulse wave sensor 20 with respect to the radial artery 30 is inappropriate, is displayed on the display/record device 34, and in Step S16 the ON/OFF switch (not shown) is placed in the OFF position to deactivate the apparatus. Thus, the apparatus easily judges whether or not the pulse wave sensor 20 is appropriately positioned on the body surface 12 with respect to the radial artery 30. In other words, the present apparatus prevents the pulse wave sensor 20 from being located on the body surface 12 at an inappropriate position where the amplitude of pulse wave detected by the pulse wave sensor 20 is relatively largely changed due to a relatively small change in the pulse wave sensor pressing force.

Since in the present embodiment the pulses detected by the pulse wave sensor 20 (pressure sensing elements 29) are subjected to the median filter treatment, the first and second force ranges Pp and Pd are determined with high precision, and accordingly the optimum pressing force Pa is determined in a reliable manner.

While the present invention has been described in its presently preferred embodiment, it is to be understood that the present invention is by no means limited to the particular details of the embodiment but may be otherwise embodied.

For example, in the event that it is judged in Step S20 of the flow chart of Fig. 3 that the value ΔPc is greater than 100 mmHg, it is possible to judge in an additional step whether or not the value ΔPc is greater than another predetermined value greater than 100 mmHg and determine the optimum pressing force Pa in each of the respective ways corresponding to the affirmative and negative judgements in the additional step. Alternatively, Steps S20 and S22 may be omitted, namely, the middle value of the overlap portion Pc may be determined as the optimum pressing force Pa irrespective of the value ΔPc.

Although, in the illustrated embodiment, in Step S22 or S25 the optimum pressing force Pa is determined by adding 50 mmHg, one second of 100 mmHg, to the minimum value Pcmin or Ppmin of the overlap portion Pc or first force range Pp, respectively, it is possible to add a value greater or smaller than 50 mmHg to the minimum value Pcmin or Ppmin.

Furthermore, in the event that the second force range Pd is zero, it is possible to effect additional steps similar to Steps S15 and S16 provided for the event that the first force range Pp is zero.

Although in the illustrated embodiment the optimum pressing force Pa is determined based on the pulse wave signals SM which are produced as the air pressure P in the pressure chamber 22 is increased, it is possible to use pulse wave signals SM which are produced as the air pressure P is decreased, for the determination of the optimum pressing force Pa.

The multiplicity of pressure sensing elements 29 may be replaced by a single pressure sensing element. In addition, the individual pressure sensing elements 29 may be constituted by various elements different from the diodes or other pressure sensing semiconductor elements.

While in the illustrated embodiment the pulse wave sensor 20 is adapted to be pressed against the radial artery 30, it is possible to use a pulse wave sensor adapted to be pressed against an arterial vessel different from the radial artery 30, such as dorsal pedal artery.

## Claims

1. A pulse wave detecting apparatus for detecting a pulse wave produced from an arterial vessel of a subject, comprising: a pulse wave sensor (20) having a press surface (28) on which at least one pressure sensing element (29) is provided, said pulse wave sensor to be pressed against an arterial vessel (30) of a subject (16) via a body surface (12) above said arterial vessel so that said at least one pressure sensing element detects a pulse wave produced from said arterial vessel of said subject;

   pressing means (10, 18, 22-26, 32) for pressing said pulse wave sensor against said arterial vessel via said body surface, while varying the pressing force over a predetermined force range;

   amplitude determining means (32, S8) for determining the amplitude of each of the pulses of the pulse wave which are detected by said at least one pressure sensing element while the pressing force of said pressing means is varied;

   minimum value determining means (32, S7) for determining a minimum value of said each pulse of the pulse wave detected by said at least one pressure sensing element;

   first and second pressing force range determining means (32, S13, S17);

   optimum pressing force determining means (32, S20-S25) for determining an optimum value of said pressing force; and

   means (32, S26) for adjusting said pressing means to said optimum value of said pressing force and obtaining a pulse wave of said subject through said pulse wave sensor pressed against said arterial vessel with the optimum pressing force, the apparatus being characterised in that

the first pressing force range determining means (32, S9-S13) is adapted to determine, based on the pulse amplitudes determined by said amplitude determining means, a first force range (Pp) of said pressing force within which the change of said pulse amplitudes is smaller than a first predetermined value, said first force range including a point at which a rate of change of said pulse amplitudes with respect to said pressing force is zero;

the second pressing force range determining means (32, S17) is adapted to determine, based on the pulse minimum values determined by said minimum value determining means, a second force range (Pd) of said pressing force within which the change of said pulse minimum values is smaller than a second predetermined value, said second force range including a point at which a rate of change of said pulse minimum values with respect to said pressing force is minimum;

optimum pressing force range determining means (32, S18, S19) is provided for determining, as an optimum force range (Pc, Pp) of said pressing force, an overlap portion (Pc) of said first and second force ranges in the event that neither said first force range (Pp) nor said second force range (Pd) is zero; and

the optimum pressing force determining means (32, S20-S25) is adapted to determine said optimum value ($\underline{Pa}$) of said pressing force such that said optimum value falls within said optimum force range.

2. The apparatus as set forth in claim 1, wherein, in the event that a value $\Delta Pc$ obtained by subtracting the lower limit Pcmin of said overlap potion (Pc) from the upper limit thereof is smaller than a first predetermined value S, said optimum pressing force determining means (32, S18-S25) determines said optimum pressing force Pa according to the following expression: Pa = Pcmin + $\Delta Pc/2$, and, in the event that said value $\Delta Pc$ is not smaller than said first predetermined value S, said optimum pressing force determining means determines said optimum value of the pressing force Pa according to the following expression: Pa = Pcmin + S/2.

3. The apparatus as set forth in any preceding claim wherein, in the event that said first force range (Pp) is not zero and said second force range (Pd) is zero, said optimum pressing force range determining means (32, S18-S25) determines said first force range as said optimum force range.

4. The apparatus as set forth in claim 3, wherein, in the event that a value $\Delta Pp$ obtained by subtracting the lower limit Ppmin of said first force range from the upper limit thereof is smaller than a second predetermined value T, said optimum pressing force determining means (32, S18-S25) determines said optimum value Pa of the pressing force according to the following expression: Pa = Ppmin + $\Delta Pp/2$, and, in the event that said value $\Delta Pp$ is not smaller than said second predetermined value T, said optimum pressing force determining said optimum pressing force Pa according to the following expression: Pa = Ppmin + T/2.

5. The apparatus as set forth in any preceding claim further comprising:

means (32, S14-S16) for, in the event that said first force range (Pp) is zero, informing an operator that a position of said pulse wave sensor (20) on said body surface (12) with respect to said arterial vessel (30) is inappropriate, and causing the pulse wave detecting apparatus to be deactivated.

6. The apparatus as set forth in any preceding claim wherein said pressing means comprises:

a housing (10);

a diaphragm (18) fluid-tightly secured to inner surfaces of said housing, said diaphragm cooperating with said housing to define a fluid-tight pressure chamber (22), said pulse wave sensor (20) being secured to an outer surface of said diaphragm so that said pulse wave sensor is movable together with said diaphragm relative to said housing;

supply means (24) for supplying said pressure chamber with a pressurized fluid so as to press said pulse wave sensor against said arterial vessel (30) via said body surface (12); and

pressure regulating means (26) for regulating said pressurized fluid supplied to said pressure chamber and thereby adjusting a fluid pressure in said pressure chamber,

said pressing force of said pressing means with which said pulse wave sensor is pressed against said arterial vessel being varied as said fluid pressure in said pressure chamber is varied by said pressure regulating means.

7. The apparatus as set forth in any preceding claim wherein said pulse wave sensor (20) comprises:

a semiconductor chip (28) of monocrystalline silicon; and

a plurality of pressure sensing diodes (29) provided in a row on one surface (28) of said semiconductor chip, said one surface of said semiconductor chip serving as said press surface of said pulse wave sensor, each of said plurality of pressure sensing diodes detecting a pulse wave produced from said arterial vessel (30).

8. The apparatus as set forth in any preceding claim wherein said at least one pressure sensing element comprises a plurality of pressure sensing elements (29), and wherein (a) said amplitude determining means (32, S8) determines an amplitude of each of pulses of the pulse wave which are detected by each of said plurality of pressure sensing elements while said pressing force of said pressing means (10, 18, 22-26) is varied, (b) said first pressing force range determining means (32, S9-S13) selects as an optimum pressure sensing element (29A) one of said plurality of pressure sensing elements which element has detected a pulse whose amplitude is the greatest of the amplitudes of the pulses detected by said plurality of pressure sensing elements, and determines said first force range (Pp) based on the amplitudes of the pulses detected by said optimum pressure sensing element, (c) said minimum value determining means (32, S7) determines an minimum value of each of the pulses detected by said optimum pressure sensing element, and (d) said second force range determining means (32, S17) determines said second force range (Pd) based on the pulse minimum values determined by said minimum value determining means.

9. The apparatus as set forth in claim 8, wherein said plurality of pressure sensing elements comprise at least three pressure sensing elements (29) provided in a row on said press surface (28) of said pulse wave sensor (20), the apparatus further comprising:

means (32, S14-S16) for, in the event that said first pressing force range determining means (32, S9-S13) selects as said optimum pressure sensing element one of two pressure sensing elements respectively positioned at opposite ends of said row, discarding the pressure sensing element selected by said first pressing force range determining means, and reselecting as said optimum pressure sensing element (29A) another pressure sensing element which has detected a pulse whose amplitude is the greatest of the amplitudes of the pulses detected by said at least three pressure sensing elements except the discarded pressure sensing element.

10. The apparatus as set forth in any preceding claim further comprising:

means (34) for displaying and recording said pulse wave detected through said pulse wave sensor (20) pressed with said optimum pressing force.

11. The apparatus as set forth in any preceding claim further comprising;

means (32) for determining a blood pressure of said subject based on said pulse wave detected through said pulse wave sensor (20) pressed with said optimum pressing force.

**Patentansprüche**

1. Puls- Wellen- Meßgerät zur Messung einer Pulswelle, die von einem arteriellen Gefäß einer Person erzeugt wird, mit: einem Puls- Wellen- Sensor (20) mit einer Druckoberfläche (28), auf der wenigstens ein Druckfühler (29) vorgesehen ist, wobei der Puls- Wellen- Sensor gegen ein arterielles Gefäß (30) einer Person (16) über eine Körperoberfläche (12) über dem arteriellen Gefäß zu drücken ist, so daß wenigstens ein Druckfühler eine von dem arteriellen Gefäß der Person erzeugte Pulswelle feststellt;

Andrückmitteln (10, 18, 22 bis 26, 32) zum Andrücken des Puls- Wellen- Sensors über die Körperoberfläche gegen das arterielle Gefäß, während die Andrückkraft in einem vorbestimmten Kraftbereich verändert wird;

Amplitudenbestimmitteln (32, S8), die die Amplitude eines jeden Pulses der Pulswelle ermitteln, die von dem wenigstens einfach vorgesehenen Druckfühler festgestellt wird, während die Andrückkraft der Andrückmittel verändert wird;

Minimalwert- Bestimmitteln (32, S7), die einen Minimalwert eines jeden Pulses der von dem wenigstens einfach vorgesehenen Druckfühler festgestellten Pulswelle ermitteln;

einem ersten und einem zweiten Andrückkraftbereich-Bestimmittel (32, S13, S17);

einem Optimalandrückkraftbereich- Bestimmittel (32, S20 bis S25) zur Ermittlung eines Optimaldruckes der Andrückkraft; und mit

Mitteln (32, S26) zur Einstellung der Andrückmittel auf den Optimalwert der Andrückkraft und zur Erzeugung einer Pulswelle der Person durch den Puls- Wellen- Sensor, der mit der optimalen Andrückkraft gegen das arterielle Gefäß gedrückt wird,
**dadurch gekennzeichnet,**

daß das erste Andrückkraftbereich- Bestimmittel (32, S9 bis S13) auf der Grundlage der von den Amplitudenbestimmitteln ermittelten Pulsamplituden einen ersten Kraftbereich (Pp) der Andrückkraft bestimmt, in dem die Änderung der Pulsamplituden kleiner als ein vorbestimmter erster Wert ist, wobei der erste Kraftbereich einen Punkt enthält, bei dem ein auf die Andrückkraft bezogener Gradient der Impulsamplituden gleich Null ist;

daß das zweite Andrückkraftbereich- Bestimmittel (32, S17) auf der Grundlage der von den Minimalwert- Bestimmitteln ermittelten Pulsminimalwerte einen zweiten Kraftbereich (Pd) der Andrückkraft bestimmt, in dem die Änderung der Puls- Minimalwerte kleiner als ein zweiter vorbestimmter Wert ist, wobei der zweite Kraftbereich einen Punkt enthält, bei dem ein auf die Andrückkraft bezogener Gradient der Puls- Minimalwerte ein Minimum hat;

daß ein Bestimmittel (32, S18, S19) für den optimalen Andrückkraftbereich vorgesehen ist, um einen Überlappungsabschnitt (Pc) des ersten und zweiten Bereichs als einen optimalen Kraftbereich (Pc, Pp) der Andrückkraft festzulegen, falls weder der erste Kraftbereich (Pp) noch der zweite Kraftbereich (Pd) gleich Null ist; und dadurch,

daß das Bestimmittel (32, S20 bis S25) den Optimalwert (Pa) der Andrückkraft derart ermittelt, daß dieser Optimalwert in den optimalen Kraftbereich fällt.

2. Gerät nach Anspruch 1, bei dem unter der Voraussetzung, daß ein durch Subtraktion der unteren Grenze Pcmin des Überlappungsabschnitts (Pc) von dessen oberer Grenze gewonnener Wert $\Delta$Pc kleiner als ein erster vorbestimmter Wert S ist, wobei das Optimalandrückkraft- Bestimmittel (32, S18 bis S25) die optimale Andrückkraft Pa gemäß der Gleichung Pa = Pcmin + $\Delta$Pc/2 ermittelt und im Falle, daß der Wert $\Delta$Pc nicht kleiner als der erste vorbestimmte Wert S ist, das Optimalandrückkraft-Bestimmittel den Optimalwert der Andrückkraft Pa nach der Gleichung Pa = Pcmin + S/2 bestimmt.

3. Gerät nach einem der vorstehenden Ansprüche, bei dem das Optimalandrückkraft- Bestimmittel (32, S18 bis S25) den ersten Kraftbereich als optimalen Kraftbereich festlegt, falls der erste Kraftbereich (Pp) nicht Null und der zweite Kraftbereich (Pd) Null ist.

4. Gerät nach Anspruch 3, dessen Optimalandrückkraft-Bestimznittel (32, S18 bis S25) den Optimalwert Pa der Andrückkraft entsprechend der Gleichung Pa = Ppmin + $\Delta$Pp/2 bestimmt, falls ein durch Subtraktion der unteren Grenze Ppmin des ersten Kraftbereichs von deren oberer Grenze gewonnener Wert $\Delta$Pp kleiner als ein zweiter vorbestimmter Wert T ist, und wenn der Wert $\Delta$Pp nicht kleiner als der zweite vorbestimmte Wert T ist, erfolgt die Optimalkraftbestimmung der optimalen Andrückkraft Pa nach der Gleichung Pa = Ppmin + T/2.

5. Gerät nach einem der vorstehenden Ansprüche, mit:
Mitteln (32, S14 bis S16), die im Falle, daß der erste Kraftbereich (Pp) Null ist, einerseits eine Bedienperson darüber informieren, daß eine Position des Puls- Wellen- Sensors (20) auf der Körperoberfläche (12) in Hinsicht auf das arterielle Gefäß (30) ungeeignet ist, und die andrerseits die Abschaltung des Puls- Wellen- Meßgerätes bewirken.

6. Gerät nach einem der vorstehenden Ansprüche, dessen Andrückmittel ausgestattet sind mit:
einem Gehäuse (10);
einer Membran (18), die zur den Innenflächen des Gehäuses sicher abgedichtet ist, wobei die Membran zusammen mit dem Gehäuse eine abgedichtete Druckkammer (22) bildet, wobei der Puls- Wellen- Sensor (20) an der Außenfläche der Membran befestigt ist, so daß der Puls- Wellen- Sensor gemeinsam mit der Membran relativ zum Gehäuse beweglich ist;
Versorgungsmitteln (24) zur Beschickung der Druckkammer mit einem Druckfluid, das den Puls- Wellen- Sensor gegen das arterielle Gefäß (30) über die Körperoberfläche (12) drückt; und mit
Druckreguliermitteln (26), die das die Druckkammer beschickende Druckfluid regulieren, wodurch ein Fluiddruck in der Kammer eingestellt wird,
wobei die die Andrückkraft der den Puls- Wellen- Sensor gegen das arterielle Gefäß drückenden Andrückkraftmittel entsprechend dem Fluiddruck in der Druckkammer von den Druckreguliermitteln variiert wird.

7. Gerät nach einem der vorstehenden Ansprüche, dessen Puls- Wellen- Sensor (20) zusammengesetzt ist aus:
einem Halbleiterchip (28) aus einkristalligem Silizium; und

einer Vielzahl von Druckfühldioden (29), die in einer Reihe auf einer Oberfläche (28) des Halbleiterchips vorgesehen sind, wobei eine Oberfläche des Halbleiterchips als Druckoberfläche des Puls- Wellen- Sensors dient, wobei jede einzelne der Vielzahl von Druckfühldioden eine Pulswelle feststellt, die von dem arteriellen Gefäß (30) erzeugt wird.

8. Gerät nach einem der vorstehenden Ansprüche, dessen wenigstens einfach vorhandener Druckfühler eine Vielzahl von Druckfühlern (29) enthält und (a) dessen Amplitudenbestimmittel (32, S8) eine Amplitude eines jeden Pulses der Pulswelle bestimmt, die von jeder einzelnen der Vielzahl von Druckfühlern festgestellt wird, während die Andrückkraft der Andrückmittel (10, 18, 22 bis 26) verändert wird, (b) dessen erstes Andrückkraftbereich- Bestimmittel (32, S9 - S13) als Optimaldruckfühler (29A) einen aus der Vielzahl von Druckfühlern aussucht, der einen Puls feststellt, desssen Amplitude die größte der von der Vielzahl der Druckfühler festgestellten Amlituden ist, und das den ersten Kraftbereich (Pp) auf der Grundlage der Amplituden der von dem Optimaldruckfühler festgestellten Amplituden festlegt, (c) dessen Minimalwert- Bestimmittel (32, S7) einen Minimalwert von einem jeden der von den Optimaldruckfühlern festgestellten Pulsen ermittelt, und (d) dessen zweites Kraftbereich- Bestimmittel (32, S17) ermittelt den zweiten Kraftbereich (Pd) auf der Grundlage der von den Minimalwert- Bestimmitteln festgestellten Pulsminimalwerte.

9. Gerät nach Anspruch 8, dessen Vielzahl von Druckfühlern wenigstens drei Druckfühler (29) in einer Reihe auf der Druckoberfläche (28) des Puls- Wellen- Sensors (20) umfasst, wobei das Gerät des weiteren aussgestattet ist mit:

Mitteln (S14 bis S16), die im Falle, daß das erste Andrückkraftbereich- Bestimmittel (32, S9 bis S13) als Optimaldruckfühler einen von zwei Druckfühlern aussucht, die sich jeweils an gegenüberliegenden Enden der Reihe befinden, die von dem ersten Andrückkraftbereich- Bestimmittel ausgesuchten Druckfühler ausrangieren und als den Optimaldruckfühler (29A) ein anderes Druckelement neu aussuchen, das Pulse festgestellt hat, deren Amlitude die größte der Amplituden der von den wenigstens drei Druckfühlern außer dem ausrangierten Druckfühler ist.

10. Gerät nach einem der vorstehenden Ansprüche, das des weiteren aussgestattet ist mit:

Mitteln (34) zur Anzeige und zur Aufzeichnung der durch den mit der optimalen Andruckkraft angedrückten Puls- Wellen- Sensor (20) festgestellten Pulswelle.

11. Gerät nach einem der vorstehenden Ansprüche, das des weiteren aussgestattet ist mit:

Mitteln (32) zur Feststellung des Blutdrucks einer Person auf der Grundlage der durch den mit der optimalen Andruckkraft angedrückten Puls- Wellen- Sensor (20) festgestellten Pulswelle.

**Revendications**

1. Appareil de détection d'onde pulsée pour détecter une onde pulsée produite par un vaisseau artériel d'un sujet, comprenant :

un capteur d'onde pulsée (20) ayant une surface de pressage (28) sur laquelle est prévu au moins un élément de détection de pression (29), ledit capteur d'onde pulsée étant pressé contre un vaisseau artériel (30) d'un sujet (16) par l'intermédiaire d'une surface du corps (12) située au-dessus dudit vaisseau artériel de sorte que ledit au moins un élément de détection de pression détecte une onde pulsée produite par ledit vaisseau artériel dudit sujet ;

des moyens de pressage (10,18,22-26,32) pour presser ledit capteur d'onde pulsée contre ledit vaisseau artériel par l'intermédiaire de ladite surface du corps, tout en faisant varier la force de pressage dans une plage de force de pressage prédéterminée ;

des moyens de détermination d'amplitude (32,S8) pour déterminer l'amplitude de chacune des impulsions de l'onde pulsée qui sont détectées par ledit au moins un élément de détection de pression pendant qu'on fait varier la force de pressage desdits moyens de pressage ;

des moyens de détermination de valeur minimale (32,S7) pour déterminer une valeur minimale de chaque dite impulsion de l'onde pulsée détectée par ledit au moins un élément de détection de pression ;

des moyens de détermination d'une première et d'une deuxième plages de force de pressage (32,S13,S17) ;

des moyens de détermination de force de pressage optimale (32,S20-S25) pour déterminer une valeur optimale de ladite force de pressage ; et

des moyens (32,S26) pour ajuster lesdits moyens de pressage à ladite valeur optimale de ladite force de pressage et obtenir une onde pulsée dudit sujet par l'intermédiaire dudit capteur d'onde pulsée pressé contre ledit vaisseau artériel avec la force de pressage optimale; l'appareil étant caractérisé en ce que :

les moyens de détermination de première plage de force de pressage (32,S9-S13) sont prévus pour déterminer, sur la base des amplitudes d'impulsion déterminées par lesdits moyens de détermination d'amplitude, une première plage de force (Pp) de ladite force de pressage à l'intérieur de laquelle la variation desdites amplitudes d'impulsion est plus petite qu'une première valeur prédéterminée, ladite première plage de force incluant un point auquel une vitesse de variation desdites amplitudes d'impulsion en fonction de ladite force de pressage est nulle ;

les moyens de détermination de deuxième plage de force de pressage (32,S17) sont prévus pour déterminer, sur la base des valeurs minimales d'impulsion déterminées par lesdits moyens de détermination de valeur minimale, une deuxième plage de force (Pd) de ladite force de pressage à l'intérieur de laquelle la variation desdites valeurs minimales d'impulsion est plus petite qu'une deuxième valeur prédéterminée, ladite deuxième plage de force incluant un point auquel une vitesse de variation des dites valeurs minimales d'impulsion en fonction de ladite force de pressage est minimale ;

les moyens de détermination de plage de force de pressage optimale (32,S18,S19) sont prévus pour déterminer, comme plage de force optimale (Pc,Pp) de ladite force de pressage, une partie en chevauchement (Pc) des dites première et deuxième plages de force dans le cas où ni ladite première plage de force (Pp) ni ladite deuxième plage de force (Pd) n'est nulle ; et

les moyens de détermination de force de pressage optimale (32,S20-S25) sont prévus pour déterminer la dite valeur optimale (Pa) de ladite force de pressage de sorte que ladite valeur optimale tombe à l'intérieur de ladite plage de force optimale.

2. Appareil suivant la revendication 1, dans lequel, dans le cas où une valeur ∆Pc obtenue en soustrayant la limite inférieure Pcmin de ladite partie en chevauchement (Pc) de sa limite supérieure est plus petite qu'une première valeur prédéterminée S, les dits moyens de détermination de force de pressage optimale (32 ,S18-S25) déterminent ladite force de pressage optimale Pa conformément à l'expression suivante : Pa = Pcmin + ∆Pc/2, et, dans le cas où ladite valeur ∆Pc n'est pas plus petite que ladite première valeur prédéterminée S, lesdits moyens de détermination de force de pressage optimale déterminent ladite valeur optimale de la force de pressage Pa conformément à l'expression suivante : Pa = Pcmin + S/2.

3. Appareil suivant une quelconque des revendications précédentes dans lequel, dans le cas où ladite première plage de force (Pp) n'est pas nulle et ladite deuxième plage de force (Pd) est nulle, lesdits moyens de détermination de plage de force de pressage optimale (32, S18-S25) déterminent ladite première plage de force comme dite plage de force optimale.

4. Appareil suivant la revendication 3, dans lequel, dans le cas où une valeur ∆Pp obtenue en soustrayant la limite inférieure Ppmin de ladite première plage de force de sa limite supérieure, est plus petite qu'une deuxième valeur prédéterminée T, lesdits moyens de détermination de force de pressage optimale (32,S18-S25) déterminent ladite valeur optimale Pa de la force de pressage conformément à l'expression suivante : Pa = Ppmin + ∆Pp/2, et, dans le cas où ladite valeur ∆Pp n'est pas plus petite que ladite deuxième valeur prédéterminée T, lesdits moyens de détermination de force de pressage optimale déterminent ladite force de pressage optimale Pa conformément à l'expression suivante : Pa = Ppmin + T/2.

5. Appareil suivant une quelconque des revendications précédentes, comprenant en outre :

des moyens (32,S14-S16) pour informer un opérateur, dans le cas où ladite première plage de force (Pp) est nulle, qu'une position dudit capteur d'onde pulsée (20) sur ladite surface du corps (12) par rapport audit vaisseau artériel (30) est inappropriée, et provoquer la désactivation de l'appareil de détection d'onde pulsée.

6. Appareil suivant une quelconque des revendications précédentes,dans lequel lesdits moyens de pressage comprennent :

un boîtier (10) ;

une membrane (18) fixée de façon étanche aux fluides aux surfaces intérieures dudit boîtier, ladite membrane coopérant avec ledit boîtier pour définir une chambre de pression étanche aux fluides (22), ledit capteur d'onde pulsée (20) étant fixé à une surface extérieure de ladite membrane de sorte que ledit capteur d'onde pulsée peut se déplacer en même temps que ladite membrane par rapport audit boîtier ;

des moyens d'alimentation (24) pour alimenter ladite chambre de pression avec un fluide comprimé de façon à presser ledit capteur d'onde pulsée contre le dit vaisseau artériel (30) par l'intermédiaire de ladite surface du corps (12) ; et

des moyens de régulation de pression (26) pour régler ledit fluide comprimé envoyé à ladite chambre de pression et régler ainsi une pression de fluide dans ladite chambre de pression ;

ladite force de pressage desdits moyens de pressage, avec laquelle ledit capteur d'onde pulsée est pressé contre ledit vaisseau artériel, variant lorsque ladite pression de fluide dans ladite chambre de pression est modifiée par lesdits moyens de régulation de pression.

7. Appareil suivant une quelconque des revendications précédentes, dans lequel ledit capteur d'onde pulsée (20) comprend :

une puce semiconductrice (28) en silicium monocristallin ; et

une pluralité de diodes de détection de pression (29) disposées en une rangée sur une première surface (28) de ladite puce semiconductrice, ladite première surface de ladite puce semiconductrice servant de dite surface de pressage dudit capteur d'onde pulsée, chacune de ladite pluralité de diodes de détection de pression détectant une onde pulsée produite par ledit vaisseau artériel (30).

8. Appareil suivant une quelconque des revendications précédentes, dans lequel ledit au moins un élément de détection de pression comprend une pluralité d'éléments de détection de pression (29), et dans lequel (a) lesdits moyens de détermination d'amplitude (32,S8) déterminent une amplitude de chacune des impulsions de l'onde pulsée qui sont détectées par chacun de ladite pluralité d'éléments de détection de pression pendant qu'on fait varier ladite force de pressage desdits moyens de pressage (10,18,22-26), (b) lesdits moyens de détermination de première plage de force de pressage (32,S9-S13) sélectionnent, comme élément de détection de pression optimal (29A), un élément de ladite pluralité d'éléments de détection de pression qui a détecté une impulsion dont l'amplitude est la plus grande des amplitudes des impulsions détectées par ladite pluralité d'éléments de détection de pression et ils déterminent ladite première plage de force (Pp) sur la base des amplitudes des impulsions détectées par ledit élément de détection de pression optimal, (c) les dits moyens de détermination de valeur minimale (32,S7) déterminent une valeur minimale de chacune des impulsions détectées par ledit élément de détection de pression optimale,et (d) lesdits moyens de détermination de deuxième plage de force (32,S17) déterminent ladite deuxième plage de force (Pd) sur la base des valeurs minimales d'impulsion déterminées par lesdits moyens de détermination de valeur minimale.

9. Appareil suivant la revendication 8, dans lequel ladite pluralité d'éléments de détection de pression comprend au moins trois éléments de détection de pression (29) disposés en une rangée sur ladite surface de pressage (28) dudit capteur d'onde pulsée (20), l'appareil comprenant en outre :

des moyens (32,S14-S16) pour, dans le cas où les dits moyens de détermination de première plage de force de pressage (32,S9-S13) choisissent comme dit élément de détection de pression optimal un des deux éléments de détection de pression respectivement situés aux extrémités opposées de ladite rangée, rejeter l'élément de détection de pression choisi par lesdits moyens de détermination de première plage de force de pressage, et choisir à la place comme dit élément de détection de pression optimal (29A) un autre élément de détection de pression qui a détecté une impulsion dont l'amplitude est la plus grande des amplitudes des impulsions détectées par lesdits au moins trois éléments de détection de pression à l'exception de l'élément de détection de pression rejeté.

10. Appareil suivant une quelconque des revendications précédentes, comprenant en outre :

des moyens (34) pour afficher et enregistrer la dite onde pulsée détectée au moyen dudit capteur d'onde pulsée (20) pressé avec ladite force de pressage optimale.

11. Appareil suivant une quelconque des revendicaitons précédentes, comprenant en outre :

des moyens (32) pourdéterminer une pression du sang dudit sujet sur la base de ladite onde pulsée détectée par ledit capteur d'onde pulsé (20) pressé avec ladite force de pressage optimale.

FIG.1

DISPLAY/RECORD DEVICE — 34

CONTROL DEVICE — 32

SM

SI

SD

PRESSURE REGULATING VALVE — 26

PRESSURIZED FLUID SUPPLY — 24

14

14

10

12

16

18 20 30 28

22

EP 0 452 578 B1

14

# FIG.2

20

29    28

# FIG.4

PULSE AMPLITUDE A AND
PULSE MINIMUM VALUE B

B

Pd

Pc

Pp

A

Pp min    Pc min

PRESSURE P (PRESSING FORCE)

S1: SWITCH ON? — NO / YES

SLOW PRESSURE INCREASE — S2

S3: ONE PULSE DETECTED? — NO / YES

S4: $P \geqq P_1$? — NO / YES

DEFLATION — S5

MEDIAN FILTER TREATMENT — S6

PULSE MAXIMUM AND MINIMUM VALUES DETERMINED — S7

AMPLITUDE DETERMINED — S8

GREATEST AMPLITUDE DETERMINED — S9

OPTIMUM PRESSURE SENSING ELEMENT SELECTED — S10

S11: INAPPROPRIATE PRESSURE SENSING ELEMENT? — NO (A) / YES

OPTIMUM PRESSURE SENSING ELEMENT RE-SELECTED

FIRST FORCE RANGE DETERMINED — S13

(A) S12

S14: $\Delta P_p \neq 0$? — NO / YES

INDICATION OF INAPPROPRIATE POSITION OF PULSE WAVE SENSOR — S15

SWITCH OFF — S16

SECOND FORCE RANGE DETERMINED — S17

S18: $\Delta P_d \neq 0$? — NO / YES

OVERLAP PORTION Pc DETERMINED — S19

S20: $\Delta P_c > 100\,mmHg$? — NO / YES

$P_a = P_{c\,min} + \Delta P_c / 2$ — S21

$P_a = P_{c\,min} + 50\,mmHg$ — S22

S23: $\Delta P_p > 100\,mmHg$? — YES / NO

$P_a = P_{p\,min} + \Delta P_p / 2$ — S24

$P_a = P_{p\,min} + 50\,mmHg$ — S25

PRESSURE $P_a$ ESTABLISHED AND HELD — S26

FIG. 3

16